# FASCICULE DE BREVET EUROPEEN

(11) **EP 3 160 587 B1**
(45) Date de publication et mention de la délivrance du brevet: **26.09.2018**
(21) Numéro de dépôt: 15736417.5
(22) Date de dépôt: 23.06.2015
(51) Int. Cl.: A61N 5/10, B29C 67/00

(54) **PROCEDE DE PRODUCTION D'UN FANTÔME PHYSIQUE RADIOMETRIQUE D'UN ORGANISME BIOLOGIQUE ET FANTÔME PHYSIQUE PRODUIT PAR CE PROCEDE**
VERFAHREN ZUR HERSTELLUNG EINES RADIOMETRISCHEN PHYSISCHEN PHANTOMS EINES BIOLOGISCHEN ORGANISMUS UND MIT DIESEM VERFAHREN HERGESTELLTES PHYSISCHES PHANTOM
METHOD OF PRODUCING A RADIOMETRIC PHYSICAL PHANTOM OF A BIOLOGICAL ORGANISM AND PHYSICAL PHANTOM PRODUCED BY THIS METHOD

(30) Priorité: 25.06.2014 FR 1455911
(43) Date de publication de la demande: 03.05.2017
(73) Titulaire: COMMISSARIAT À L'ÉNERGIE ATOMIQUE ET AUX ÉNERGIES ALTERNATIVES, 75015 Paris (FR)
(72) Inventeur: BORDY, Jean-Marc, 91600 Savigny Sur Orge (FR)
(74) Mandataire: Gevers & Orès
(86) Numéro de dépôt international: PCT/EP2015/064126
(87) Numéro de publication internationale: WO 2015/197625

(56) Documents cités:
- KR-B1- 101 378 875
- US-A1- 2005 077 459
- RAYA R GALLAS ET AL: "An anthropomorphic multimodality (CT/MRI) phantom prototype for end-to-end tests in radiation therapy", ARXIV.ORG, CORNELL UNIVERSITY LIBRARY, 201 OLIN LIBRARY CORNELL UNIVERSITY ITHACA, NY 14853, 16 février 2014 (2014-02-16), XP080005908,
- BALDOCK C ET AL: "TOPICAL REVIEW; Polymer gel dosimetry", PHYSICS IN MEDICINE AND BIOLOGY, INSTITUTE OF PHYSICS PUBLISHING, BRISTOL GB, vol. 55, no. 5, 7 mars 2010 (2010-03-07), pages R1-R63, XP020171822, ISSN: 0031-9155
- F. RENGIER ET AL: "3D printing based on imaging data: review of medical applications", INTERNATIONAL JOURNAL OF COMPUTER ASSISTED RADIOLOGY AND SURGERY, vol. 5, no. 4, 15 mai 2010 (2010-05-15), pages 335-341, XP055139721, ISSN: 1861-6410, DOI: 10.1007/s11548-010-0476-x

## Description

La présente invention concerne un procédé de production d'un fantôme physique radiométrique représentatif d'un organisme biologique pour déterminer expérimentalement avec précision la distribution tri-dimensionnelle des doses de rayonnement reçues ou à recevoir par l'organisme.

La présente invention concerne également un fantôme physique radiométrique produit par le procédé et un système de mise en oeuvre du procédé de production du fantôme.

La présente invention concerne encore un procédé de détermination expérimentale de la distribution tri-dimensionnelle des doses de rayonnement réelles reçues ou à recevoir par l'organisme biologique à l'aide dudit fantôme physique.

Lorsqu'un traitement de radiothérapie externe est administré à un patient, les doses de rayonnement reçues doivent être vérifiées ou lorsque un accident radiologique a eu lieu, les données de reconstitution de l'accident doivent être validées.

Aujourd'hui, la vérification des traitements en radiothérapie externe est obligatoire et réalisée au travers de la dosimétrie in vivo suivant des règles fixées, par exemple celles décrites dans le document intitulé « Guide pour la pratique quotidienne de la Dosimétrie in Vivo en radiothérapie externe », rédigé par un collectif, E. Bouche et al., sous l'égide de l'Institut National du Cancer (INCa) avec la collaboration de la Société Française de Physique Médicale (SFPM) et de l'Autorité de Sureté Nucléaire.

La dosimétrie in vivo est réalisée au moyen de dosimètres qui peuvent être à lecture différée comme par exemple les dosimètres thermo-luminescents, ou à lecture instantanée comme par exemple dosimètres électroniques à diode, posés sur la peau pendant le traitement.

La dosimétrie in vivo réalisée par des dosimètres électroniques à diodes est par exemple décrite dans l'article de M. Besbes et al., intitulé « Étude de la dosimétrie in vivo par semi-conducteurs EPD-20 dans les conditions de l'irradiation corporelle totale », publié dans Cancer Radiothérapie vol 14 - n°1 - janvier 2010 pp 29-33.

La dosimétrie in vivo peut aussi être réalisée à partir d'images en deux dimensions obtenues au moyen d'un dispositif d'imagerie électronique portatif EPID (dénommé en anglais Electronic Portal Imaging Device).

Dans les deux cas de mise en oeuvre de la dosimétrie in vivo, il s'agit d'une vérification partielle des doses de rayonnement reçues, parfois réalisée a posteriori, notamment dans le cas de l'utilisation de dosimètres à lecture différée, puisqu'elle ne permet pas d'accéder à la connaissance d'une distribution tridimensionnelle (3D) des doses reçues à l'intérieur du corps du patient, sauf à utiliser des logiciels de reconstruction comme décrit dans l'article de Mathilda van Zijtveld et al., intitulé « 3D dose reconstruction for clinical evaluation of IMRT pretreatment vérification with an EPID », publié dans Radiotherapy and Oncology 82 (2007), pages 201-207..

Lorsque un accident radiologique a eu lieu, la recherche des conditions d'exposition est réalisée au moyen d'un logiciel de reconstitution comme décrit dans l'article de J.F. Bottollier-Depois et al., intitulé « Techniques de dosimétrie physique pour la reconstitution d'accident radiologique », publié dans J. Chim. Phys., Volume 95, Number 4, April 1998, pp. 685-690.

Ce type de logiciel, partant de données géométriques décrivant les lieux de l'accident, y introduit un fantôme anthropomorphique standard numérique.

Comme dans le cas précédent, la distribution des doses dans le fantôme n'est pas réellement représentative du patient car la morphologie de ce dernier diffère de celle du fantôme utilisé. En outre, les logiciels de reconstitution doivent être validés expérimentalement.

Dans les deux cas décrits ci-dessus, une autre solution consiste à irradier dans les mêmes conditions que le patient des fantômes physiques réels standards plus ou moins anthropomorphiques à l'intérieur desquels un grand nombre de dosimètres ponctuels ont été disposés. La mesure de chacun de ces dosimètres permet de reconstruire une cartographie en trois dimensions des doses de rayonnement reçues dans le fantôme. Cependant, même dans ce cas, la distribution des doses dans le fantôme n'est pas réellement représentative du patient car la morphologie particulière du patient diffère de la morphologie standard des fantômes utilisés et la reconstitution 3D est obtenue dans une « géométrie » éloignée de la réalité anthropomorphique du patient.

Raya R. Gallas et al. dans "An anthropomorphic multimodality (CT/MRI) phantom prototype for end-to-end tests in radiation therapy" propose un fantôme physique radiométrique d'une tête et un procédé et système de production correspondants.

Le problème technique est d'améliorer la précision des procédés de vérification et de validation des plans de traitement en radiothérapie externe et/ou des calculs de reconstitution des accidents radiologiques en termes de prise en compte du particularisme anatomique de chaque organisme lorsqu'un fantôme physique est utilisé.

Le problème technique est d'avoir accès à une distribution tridimensionnelle des doses de rayonnement reçues à l'intérieur d'un fantôme anthropomorphique réaliste de l'organisme, humain ou non, qui prenne en compte ses particularités anatomiques.

Le problème technique est de produire à la carte un fantôme physique radiométrique, représentatif du patient dans ses différences avec d'autres patients de la même espèce biologique.

A cette fin, l'invention a pour objet un procédé de production d'un fantôme physique radiométrique d'un organisme biologique pour déterminer expérimentalement sous des conditions d'irradiation prédéterminées une distribution volumique des doses de rayonnement reçues par le fantôme, représentative de la distribution des doses qui seraient reçues par l'organisme biologique placée dans les mêmes conditions d'irradiation, l'organisme biologique ayant au moins deux volumes de tissus biologiques dont les compositions chimiques et les masses volumiques sont nettement différentes,
le procédé de production comprenant les étapes consistant à :
à partir d'une ou de plusieurs images tridimensionnelles anatomiques des tissus en termes de compositions chimiques et de masses volumiques, déterminer un modèle tridimensionnel radiologique, représentatif de l'absorption et la diffusion du rayonnement à l'intérieur de l'organisme biologique, en regroupant dans des organes radiologiques les tissus adjacents entre eux et ayant des compositions chimiques et des masses volumiques voisines,
chaque organe radiologique occupant géométriquement dans l'espace un volume radiologique, égal ou approchant par lissage des contours de son enveloppe la somme des volumes de tous les tissus regroupés dans le même organe radiologique, et étant caractérisé par une classe radiologique identifiant la plage des compositions chimiques et des masses volumiques voisines des tissus regroupés dans le même organe radiologique ; puis
à partir du modèle radiologique, réaliser à l'aide d'une impression 3D une ossature matérielle du fantôme physique en reproduisant chaque volume radiologique par une enceinte remplie d'air et délimitée par une paroi, la paroi étant constituée d'une encre d'impression 3D; puis
remplir chaque enceinte de l'ossature matérielle du fantôme, associée à un organe radiologique par un gel dosimétrique dont les caractéristiques physiques sont proches ou identiques à celles de la classe des compositions chimiques et des masses volumiques de l'organe radiologique.

Suivant des modes particuliers de réalisation, le procédé de production d'un fantôme physique radiométrique comporte l'une ou plusieurs des caractéristiques suivantes, prises seules ou en combinaison :
.- pendant la détermination du modèle radiologique,
   pour chaque regroupement de tissus biologiques dans un même organe radiologique,
   un volume radiologique intermédiaire est d'abord déterminé comme étant égal à la somme des volumes des tissus regroupés dans l'organe radiologique, puis
   le volume radiologique de l'organe radiologique est déterminé en lissant les contours du volume radiologique intermédiaire afin de limiter les anfractuosités et faciliter le remplissage du gel dosimétriqe ;
.- le procédé de production d'un fantôme physique radiométrique comprend en outre une étape de préparation de plans logiciels de fabrication de l'ossature matérielle du fantôme et de traduction de ces plans en un programme de commandes d'impression 3D de l'ossature, insérée entre l'étape de détermination du modèle radiologique et l'étape d'impression 3D de l'ossature du fantôme, dans laquelle étape de préparation,
   pour chaque enceinte associée à un organe radiologique, le nombre des orifices de remplissage d'entrée de gel, le nombre des orifices de remplissage de sortie d'air, les emplacements et les diamètres du ou des orifices de remplissage d'entrée de gel et du ou des orifices de remplissage de sortie d'air, ainsi que les trajets et les diamètres des conduits de remplissage de l'enceinte, reliés entre les orifices de remplissage et l'extérieur du fantôme, sont déterminés de sorte
   à permettre le remplissage de l'organe radiologique sans bulles de volume ;
.- le nombre total des orifices de remplissage, les emplacements des orifices de remplissage et les trajets des conduits de remplissage de l'ensemble des enceintes sont déterminés pour ne pas gêner la propagation du rayonnement vers une zone à traiter en fonction de l'emplacement de la zone à traiter et/ou de l'angle d'entrée du faisceau de rayonnement ;
.- le nombre total des orifices de remplissage, les emplacements des orifices de remplissage et les trajets des conduits de remplissage, les formes lissées des volumes des enceintes sont déterminés pour ne pas gêner la propagation du rayonnement vers une zone à traiter en fonction de l'emplacement de la zone à traiter et/ou de l'angle d'entrée du faisceau de rayonnement ;
.- les gels dosimétriques sont compris dans l'ensemble formé par les gels de Fricke et les gels polymère.

L'invention a également pour objet un procédé de détermination expérimentale d'une distribution volumique de doses de rayonnement reçues par un fantôme physique radiométrique représentative d'une distribution de doses qui seraient reçues par la partie ou de la totalité d'un organisme biologique placé dans de mêmes conditions d'irradiation prédéterminées, comprenant les étapes consistant à :
fournir un fantôme physique rempli de ses gels dosimétriques et fabriqué selon le procédé défini ci-dessus;
irradier ensuite le fantôme physique sous les conditions d'irradiation prédéterminées ;
procéder à une lecture d'un paramètre physique des gels in situ dans le fantôme en plaçant le fantôme rempli des gels irradiés dans un appareil d'imagerie utilisant par exemple un procédé par transmission optique ou dans un appareil d'imagerie RMN et l'encoder sous forme de données brutes de lecture ;

Traiter ensuite les données brutes de lecture pour déterminer une image de la distribution tridimensionnelle des doses de rayonnement reçues dans la partie ou la totalité de l'organisme biologique.

Suivant des modes particuliers de réalisation, le procédé de détermination expérimentale d'une distribution volumique de doses de rayonnement reçues par un fantôme comporte l'une ou plusieurs des caractéristiques suivantes, prises seules ou en combinaison :
.- les informations brutes de lecture représentent un paramètre physique lu compris dans l'ensemble formé par une densité optique, un temps de relaxation ;
.- l'étape de traitement des données de lecture comprend les étapes consistant à
   traduire l'information brute de lecture en termes de doses absorbées ; et
   regrouper les données traduites en termes de doses absorbées pour créer des images particulières comprises dans l'ensemble des coupes bidimensionnelles selon un plan de coupe prédéterminé, des surfaces « isodose » en trois dimensions, des images proposées par les logiciels de planification de traitement de radiothérapie ;
.- les rayonnements sont des rayonnements de particules comprises dans l'ensemble formé par les photons, les électrons, les protons, les noyaux lourds..

L'invention a également pour objet un fantôme physique radiométrique d'un organisme biologique pour déterminer expérimentalement sous des conditions d'irradiation prédéterminées une distribution volumique des doses de rayonnement reçues par le fantôme représentative de la distribution des doses qui seraient reçues par l'organisme biologique placée dans les mêmes conditions d'irradiation, l'organisme biologique ayant au moins deux volumes de tissus biologiques dont les compositions chimiques et les masses volumiques sont nettement différentes, caractérisé en ce que le fantôme physique comprend
au moins deux enceintes maintenues en position dans un cadre de support, délimitées par des parois respectives constituées d'encre solide d'impression 3D transparente au rayonnement, et remplie chacune d'un gel dosimétrique différent,
les au moins deux enceintes ayant des volumes radiologiques différents et séparés, chaque volume radiologique étant identique ou s'approchant par lissage des contours de la somme des volumes d'un ensemble complet de tissus biologiques regroupés de l'organisme biologique imité radiologiquement, lesdits tissus regroupés étant adjacents entre eux et ayant des compositions chimiques et des masses volumiques voisines, et
les au moins deux gels dosimétriques, remplissant chacun au moins une enceinte différente ayant chacun des caractéristiques physiques proches des compositions chimiques et des masses volumiques des tissus biologiques adjacents regroupés dont la somme des volumes est identique ou est approchée par lissage du volume radiologique de l'enceinte que le gel remplit.

Suivant des modes particuliers de réalisation, le fantôme physique radiométrique comporte l'une ou plusieurs des caractéristiques suivantes, prises seules ou en combinaison :
.- chaque enceinte comporte au moins un orifice de remplissage d'entrée de gel et au moins un orifice de remplissage de sortie d'air, et au moins deux conduits de remplissage reliés entre les orifices de remplissage et l'extérieur du fantôme, et
   pour chaque enceinte, le nombre des orifices de remplissage d'entrée de gel, le nombre des orifices de remplissage de sortie d'air, les emplacements et les diamètres du ou des orifices de remplissage d'entrée de gel et du ou des orifices de remplissage de sortie d'air, ainsi que les trajets et les diamètres des conduits de remplissage de l'enceinte, reliés entre les orifices de remplissage et l'extérieur du fantôme, sont configurés pour permettre le remplissage de l'enceinte à un débit prédéterminé sans création de bulle de volume.

L'invention a également pour objet un système de production d'un fantôme physique radiométrique simulant les propriétés d'absorption ou de diffusion d'un rayonnement d'un organisme biologique, l'organisme biologique ayant au moins deux volumes de tissus biologiques dont les compositions chimiques et les masses volumiques sont nettement différentes,
le système comprenant
un moyen de fourniture d'une ou de plusieurs images tridimensionnelles anatomiques des tissus en termes de compositions chimiques et de masses volumiques,
un calculateur électronique configuré pour
à partir d'une ou de plusieurs images tridimensionnelles anatomiques des tissus en termes de compositions chimiques et de masses volumiques, déterminer un modèle tridimensionnel radiologique, représentatif de l'absorption et la diffusion du rayonnement à l'intérieur de l'organisme biologique, en regroupant dans des organes radiologiques les tissus adjacents entre eux et ayant des compositions chimiques et des masses volumiques voisines,
chaque organe radiologique occupant géométriquement dans l'espace un volume radiologique, égal ou approchant par lissage des contours de son enveloppe la somme des volumes de tous les tissus regroupés dans le même organe radiologique, et étant caractérisé par une classe radiologique identifiant la plage des compositions chimiques et des masses volumiques voisines des tissus regroupés dans le même organe radiologique, et
à partir du modèle tridimensionnel radiologique déterminer les plans d'une ossature matérielle du fantôme comme étant un ensemble d'enceintes, de conduits de remplissage en gel des enceintes, et d'éléments de support pour maintenir en position les enceintes entre elles, puis traduire les plans de l'ossature matérielle en un programme de commandes d'impression 3D à exécuter par une imprimante 3D prédéterminée,
l'imprimante 3D prédéterminée configurée pour réaliser l'ossature matérielle du fantôme non rempli des gels dosimétriques, à l'aide d'encres d'impression 3D transparentes au rayonnement ;
au moins deux gels dosimétriques différents destinés à remplir chacun une enceinte radiologique différente associée à un organe radiologique différent en termes de classe de compositions chimiques et de masses volumiques, et
un moyen de remplissage du fantôme vide par les aux moins deux gels dosimétriques.

Suivant des modes particuliers de réalisation, le système de production d'un fantôme physique radiométrique comporte l'une ou plusieurs des caractéristiques suivantes, prises seules ou en combinaison :
.- le calculateur électronique est configuré pour chaque regroupement de tissus biologiques dans un organe radiologique,
   déterminer un volume radiologique intermédiaire comme étant égal à la somme des volumes des tissus regroupés dans l'organe radiologique, et
   déterminer le volume radiologique de l'organe radiologique en lissant les contours du volume radiologique intermédiaire afin de limiter les anfractuosités et faciliter le remplissage future du gel ;
.- le calculateur électronique est configuré pour chaque organe radiologique,
   déterminer le nombre des orifices de remplissage d'entrée de gel, le nombre des orifices de remplissage de sortie d'air, chaque emplacement et diamètre du ou des orifices de remplissage d'entrée de gel, chaque emplacement et diamètre du ou des orifices de remplissage de sortie d'air, ainsi que les trajets et les diamètres des conduits de remplissage des volumes des organes radiologiques, reliés entre les orifices de remplissage et l'extérieur du fantôme, de sorte
   à permettre le remplissage de l'organe radiologique sans bulles de volume ;
.- le calculateur électronique est configuré pour déterminer le nombre total des orifices de remplissage, les emplacements des orifices de remplissage et les trajets des conduits de remplissage de sorte à ce que les conduits ne gênent pas la propagation du rayonnement vers une zone à traiter en fonction de l'emplacement de la zone à traiter, de l'angle d'entrée du faisceau de rayonnement ;
.- le calculateur électronique est configuré pour déterminer le nombre total des orifices de remplissage, les emplacements des orifices de remplissage et les trajets des conduits de remplissage, les formes lissés des volumes des organes radiologiques pour ne pas modifier significativement la propagation du rayonnement vers une zone à traiter en fonction de l'emplacement de la zone à traiter, de l'angle d'entrée du faisceau de rayonnement.

L'invention a également pour objet un système de détermination expérimentale d'une distribution volumique de doses de rayonnement reçues par un fantôme représentative d'une distribution de doses qui seraient reçues par la partie ou de la totalité d'un organisme biologique placé dans de mêmes conditions d'irradiation prédéterminées, comprenant :
un système de production d'un fantôme physique radiométrique rempli de gels dosimétriques défini ci-dessus;
un moyen d'irradiation du fantôme physique par un rayonnement sous des conditions d'irradiation prédéterminées ;
un moyen de lecture d'un paramètre physique des gels in situ dans le fantôme lorsque ce dernier rempli des gels irradiés est placé dans un appareil d'imagerie utilisant par exemple un procédé par transmission optique ou dans un appareil d'imagerie RMN et un moyen d'encodage du paramètre physique lu sous forme de données brutes de lecture ;
un moyen de traitement des données brutes de lecture pour déterminer une image de la distribution tridimensionnelle des doses de rayonnement reçues dans l'organisme biologique.

L'invention a également pour objet un produit programme d'ordinateur comportant un ensemble d'instructions qui lorsqu'elles sont chargées et exécutées par le calculateur électronique et/ou le moyen de traitement des données brutes de lecture du système de production et du système de détermination expérimentale définis ci-dessus mettent en oeuvre une partie des étapes du procédé de production défini ci-dessus et dans le procédé de détermination expérimentale défini ci-dessus.

L'invention est définie par le jeu des revendications 1 - 19, sera mieux comprise à la lecture de la description de plusieurs formes de réalisation qui vont suivre, données uniquement à titre d'exemples et faites en se référant aux dessins dans lesquels :
La Figure 1 est un ordinogramme d'un mode de réalisation d'un procédé de production d'un fantôme physique radiométrique selon l'invention ;
La Figure 2 est un ordinogramme d'un mode de réalisation de détermination expérimentale d'une distribution volumique de doses de rayonnement reçues par un fantôme physique produit selon le procédé de la Figure 1 ;
La Figure 3 est un ensemble d'une première vue globale de dessus d'un exemple de modèle radiologique défini dans le procédé de production de la Figure 1 et d'une coupe du modèle radiologique suivant un plan de coupe médian III-III, perpendiculaire à la vue de dessus ;
La Figure 4 est une vue d'une coupe selon le même plan de coupe de la Figure 3 de l'ossature du fantôme physique obtenue après l'étape d'impression 3D du procédé de production de la Figure 1 ;
La Figure 5 est une vue d'une coupe du fantôme physique rempli des gels dosimétriques non irradiés et obtenu à la fin du procédé de production de la Figure 1, le plan de coupe étant identique à celui des Figures 3 et 4 ;
La Figure 6 est une vue de l'architecture d'un système de production d'un fantôme physique de la Figure 5 qui met en oeuvre le procédé de production de la Figure 1 ;
La Figure 7 est une architecture d'un système de détermination expérimentale d'une distribution volumique de doses de rayonnement reçues par le fantôme de la Figure 5, produit par le système de production de la Figure 6, qui met en oeuvre le procédé de détermination expérimentale de la figure 2.

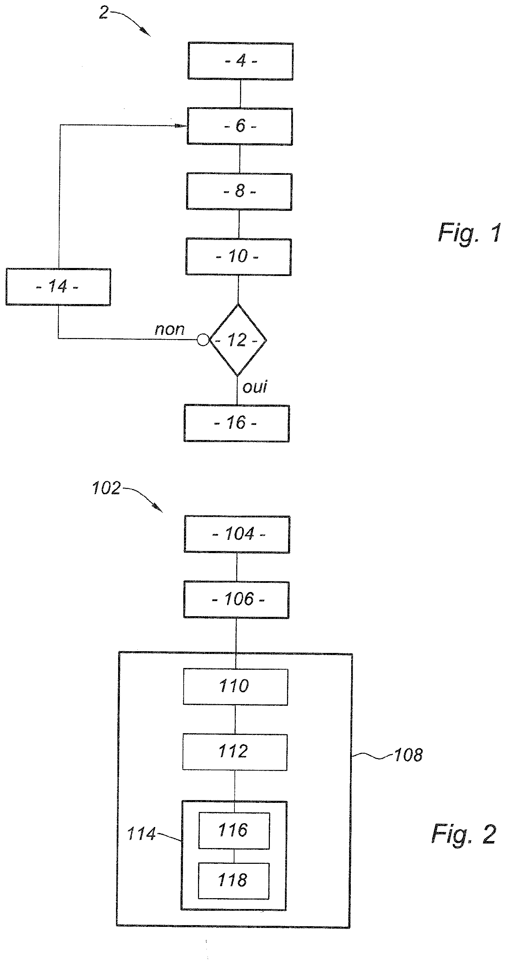

Suivant la Figure 1 et un mode de réalisation général de l'invention, un procédé de production 2 d'un fantôme physique radiométrique d'un organisme biologique comprend un ensemble d'étapes 4, 6, 8, 10, 12, 14 et 16.

Le fantôme physique radiométrique est conçu et réalisé par le procédé 2 pour simuler à la carte, c'est-à-dire de manière individualisée et la plus réaliste possible, les propriétés d'absorption et de diffusion d'un rayonnement par un organisme biologique dont on connaît avec précision les caractéristiques anatomiques propres, et pour permettre une détermination expérimentale physique sous des conditions d'irradiation prédéterminées d'une distribution volumique des doses de rayonnement reçues, représentative, au sens de pratiquement identique, de la distribution des doses de rayonnement qui seraient reçues par ou qu'auraient reçues l'organisme biologique placé dans les mêmes conditions d'irradiation.

L'organisme biologique est supposé ici suffisamment complexe pour posséder au moins deux volumes compacts et séparés de tissus biologiques différents dont les compositions chimiques et les masses volumiques sont nettement différentes, par exemple l'os ou le poumon.

Dans la première étape 4, une ou plusieurs images tridimensionnelles anatomiques des tissus biologiques de l'organisme biologique sont fournies. La ou les images tridimensionnelles anatomiques représentent les compositions chimiques et les masses volumiques des tissus biologiques.

La ou les images tridimensionnelles anatomiques sont par exemple des images diagnostic en 3D représentant l'anatomie interne et externe d'un patient.

Puis, dans la deuxième étape 6, à partir de la ou des images tridimensionnelles anatomiques des tissus biologiques fournies dans la première étape 4, un modèle tridimensionnel radiologique, représentatif de l'absorption et de la diffusion du rayonnement à l'intérieur de l'organisme biologique, est déterminé en regroupant dans des « organes radiologiques » les tissus adjacents entre eux ayant des compositions chimiques et des masses volumiques identiques ou voisines.

Chaque organe radiologique est défini et caractérisé par un volume d'organe radiologique et une classe de compositions chimiques et de masses volumiques voisines.

Le volume d'organe radiologique d'un organe radiologique donné est défini comme un volume géométrique ou un ensemble de points, délimité par ou contenu dans une surface d'enveloppe, égal ou approchant par lissage de ses contours la somme des volumes géométriques contenant la totalité des tissus regroupés dans l'organe radiologique.

La classe d'un organe radiologique donné désigne un ensemble de couples, formés chacun par une composition chimique et une masse volumique, et voisins en terme de densité électronique. A chaque classe peut être associée une caractéristique d'identification, comme une plage distincte de masses volumiques et de densités électroniques voisines, représentatives d'un tissu à simuler.

Pour chaque regroupement de tissus biologiques dans un organe radiologique, un volume radiologique intermédiaire est déterminé comme étant égal à la somme des volumes des tissus regroupés dans l'organe radiologique, et ensuite le volume radiologique de l'organe radiologique est déterminé en lissant les contours du volume radiologique intermédiaire afin de limiter les anfractuosités et faciliter le remplissage d'un gel dosimétrique représentatif de l'organe radiologique.

En variante, l'étape de détermination du modèle radiologique est dépourvue de toute étape de lissage des contours d'un volume d'organe radiologique.

De manière pratique, lorsque l'organisme biologique est un être humain, un modèle 3D radiologique anthropomorphique le plus réaliste possible de l'anatomie interne et externe du patient, est réalisé à l'aide d'un calculateur électronique et d'un logiciel. Le logiciel met en oeuvre la deuxième étape 6 en traitant d'abord les données brutes de l'imagerie anatomique pour identifier les différents tissus biologiques à partir de l'analyse des niveaux de gris des images diagnostics, les niveaux de gris étant représentatifs de la composition chimique et de la masse volumique d'un tissu. Puis, des organes radiologiques sont déterminés par regroupement des tissus biologiques, et les contours des organes sont lissés afin de limiter les anfractuosités afin de faciliter le remplissage ultérieur par les gels dosimétriques.

Ici le vocable « organe radiologique » est utilisé pour désigner au sens large tous les volumes de tissus, regroupés ou non, susceptibles d'être imprimés par une imprimante 3D. Les tissus concernés sont par exemple ceux des os, des muscles, des poumons.

Pour les organes radiologiques ne présentant pas d'anfractuosités, comme par exemple les muscles, il s'agit d'un lissage classique pour limiter la surface totale des parois et donc réduire la quantité de matière d'encre de l'imprimante 3D utilisée pour construire les volumes des organes. Le moins de matière d'encre est utilisée, le moins on perturbe la propagation du rayonnement par rapport à la réalité, même si les encres sont choisies pour être transparentes au rayonnement.

Une alternative est d'utiliser une encre dont la composition est proche de celle des tissus, mais même dans ce cas, pour réduire le temps d'impression et la quantité de matière utilisée cette fois dans un but économique, il vaut mieux lisser au préalable la surface des parois formant les organes.

Pour des organes présentant des anfractuosités, ces dernières peuvent être « gommées » ou effacées pour aboutir à une représentation plus simple de l'organes par des formes géométriques usuelles.

Prenons l'exemple des vertèbres. L'ensemble des vertèbres est représenté par exemple par un cylindre pouvant épouser par exemple la forme d'une scoliose. La base du cylindre peut être circulaire, elliptique ou moins régulière pour être plus réaliste. Plus la colonne vertébrale est susceptible de modifier la propagation du rayonnement vers la zone à traiter ou à explorer, moins la forme des volumes peut être simplifiée. Le même raisonnement s'applique aux mâchoires, les dents pouvant être assimilées à de l'os.

S'agissant de certains organes, par exemple, les intestins, le foie, la rate, il n'est pas forcément nécessaire de les reproduire séparément en tant que plusieurs organes radiologiques distincts. La composition de ces organes peut être considérée comme suffisamment proche pour permettre une représentation groupée en un seul organe radiologique.

Comme autre exemple, la représentation des poumons doit prendre en compte la trachée et les grandes bronches mais au-delà c'est la masse volumique du gel de remplissage qui permet de reproduire en moyenne le « mélange tissu-air ».

Le degré de réalisme de l'anatomie interne dépend aussi du nombre de gels différents mis à disposition de sorte que l'on puisse individualiser des organes réputés proches par leur composition.

De manière particulière, trois gels dosimétriques sont suffisants, un premier gel pour le poumon, un deuxième gel pour les os et un troisième gel pour les tissus mous. Pour les os, il n'est pas non plus forcément nécessaire, en fonction des cas, que ceux qui n'interfèrent pas avec les faisceaux de rayonnement soient représentés.

Il est à remarquer que pour certains os, s'il n'est pas nécessaire de connaître de manière détaillée la distribution des doses de rayonnement dans de l'os, ils pourront être représentés par des volumes pleins imprimés avec une encre « équivalent os » ou approchante.

Dans la troisième étape 8, des plans logiciels de fabrication d'une ossature matérielle du fantôme sont déterminées.

Ces plans de fabrication prennent en compte un certain nombre d'exigences.

Les encres d'impression 3D, constituées de matières plastiques ou d'autres matériaux durcissables étanches aux gels, peuvent être choisies pour la similitude de leurs propriétés en termes d'interaction avec les rayonnements ionisants avec celles des gels et tissus organiques. Les encres d'impression choisies seront utilisées pour former les parois des enceintes des volumes des organes radiologiques appelées à contenir les gels dosimétriques, une structure de maintien des enceintes entre elles, et des conduits de remplissage des organes radiologiques débouchant à l'extérieur du fantôme.

Pour chaque enceinte qui matérialisera le volume d'un organe radiologique, deux conditions doivent être respectées :
- L'enceinte doit être maintenue en position au sein de l'ossature au moyen d'un support de maintien à imprimer en même temps que l'enceinte de l'organe,
- deux orifices de remplissage au moins doivent être pratiqués dans les parois de l'enceinte qui relient le volume de l'enceinte à l'extérieur du fantôme pour permettre le remplissage du volume par le gel, au moins un orifice servant d'entrée au gel dosimétrique, au moins un orifice de sortie pour permettre l'évacuation de l'air au fur et à mesure du remplissage du gel sans formation de bulle d'air.

Ainsi, dans l'étape de préparation des plans de l'ossature du fantôme physique, pour chaque organe du modèle au moins un orifice de remplissage d'entrée de gel, au moins un orifice de remplissage de sortie d'air, ainsi que des conduits de remplissage des volumes des organes radiologiques, reliés entre les orifices de remplissage et l'extérieur du fantôme, sont déterminés respectivement au travers de leurs emplacements, leurs diamètres pour les orifices de remplissage, leurs emplacements, leur trajets et leurs diamètres pour les conduits de remplissage par rapport à un repère géométrique triaxial lié à l'ossature virtuelle.

Pour chaque organe radiologique, le nombre des orifices de remplissage d'entrée de gel, le nombre des orifices de remplissage de sortie d'air, les emplacements et les diamètres de l'au moins un orifice de remplissage d'entrée de gel et de l'au moins un orifice de remplissage de sortie d'air, ainsi que les trajets et les diamètres des conduits de remplissage des volumes des organes radiologiques, reliés entre les orifices de remplissage et l'extérieur du fantôme, sont déterminés de sorte à permettre le remplissage de l'organe radiologique sans bulles de volume.

De manière supplémentaire et en variante, le nombre total des orifices de remplissage, les emplacements des orifices de remplissage et les trajets des conduits de remplissage, les formes lissés des volumes des organes radiologiques sont déterminés pour ne pas modifier significativement la propagation du rayonnement vers une zone à traiter en fonction de l'emplacement de la zone à traiter, de l'angle d'entrée du faisceau de rayonnement.

Le système de remplissage, formé par les conduits de remplissage peut servir également de système de maintien en formant une partie du support de maintien des organes radiologiques.

Le nombre total des orifices de remplissage, les emplacements des orifices de remplissage et les trajets des conduits de remplissage sont déterminés pour ne pas modifier significativement la propagation du rayonnement vers une zone à traiter en fonction de l'emplacement de la zone à traiter, de l'angle d'entrée du faisceau de rayonnement. Cela peut impliquer de connaître au moins succinctement l'angle d'entrée des faisceaux de rayonnement.

Dans le cas d'une exposition globale, les systèmes de remplissage doivent être aussi petits que possible.

Selon leur position et leur nombre, et la perturbation introduite sur la distribution des doses dans le fantôme qu'ils introduisent dans le modèle par rapport à la réalité et déterminable virtuellement par un outil de calcul, un processus de calcul itératif optimisant leur nombre et la position des conduits peut conduire à reprendre le travail au niveau du lissage des surfaces des organes ou du positionnement des orifices de remplissage.

Une fois déterminés les plans de l'architecture de l'ossature du fantôme, c'est-à-dire les plans intégrant dans une même structure les parois des enceintes munies de leurs orifices de remplissage, le système de maintien et le système de remplissage, ces plans sont traduits dans la troisième étape 8 en un programme de commandes d'impression 3D, adaptées à une imprimante 3D donnée, permettant de réaliser effectivement l'ossature matérielle du fantôme.

Dans une quatrième étape 10, l'ossature matérielle du fantôme physique dépourvue de gels, est réalisée par un procédé d'impression 3D à l'aide de l'imprimante 3D, et du programme de commandes élaborées dans la troisième étape 8.

Dans l'ossature matérielle du fantôme physique dépourvu de gels ainsi réalisée, les cavités formant les volumes des organes destinés à être remplis de gels dosimétriques sont remplies d'air, et sont délimitées chacune par des parois solides constituées par les encres de l'imprimante 3D choisies pour leurs propriétés équivalentes à celles des tissus biologiques vis-à-vis de l'interaction des rayonnements ionisants.

Ensuite, dans une cinquième étape 12 de test, la qualité de la conception et de la réalisation du fantôme physique à vide sans gel est vérifiée à travers des tests de performances mécaniques et radiologiques.

Les performances testées concernent l'absence de fuites du fantôme, l'homogénéité du remplissage, et l'absence de perturbation introduite sur la distribution des doses dans le fantôme par les orifices et les conduits de remplissage.

L'absence de fuite est vérifiée de manière suffisante par un test de remplissage en eau.

L'homogénéité du remplissage est vérifiée par l'absence de bulle d'air.

L'absence de perturbation, introduite sur la distribution des doses dans le fantôme par les orifices et les conduits de remplissage, peut être vérifiée en visualisant au moyen de faisceaux lumineux le trajet des futurs faisceaux de rayonnements ionisants.

Si au moins un des tests échoue, les anomalies et les points critiques du fantôme sont identifiés, et traduits en exigences supplémentaires de conception du fantôme dans une sixième étape 14 de définition de contraintes supplémentaires et les deuxième, troisième, quatrième, cinquième étapes, 6, 8, 10, 12 sont à nouveaux exécutées.

Si les tests des performances mécaniques et radiologiques sont remplies avec succès, alors dans une septième étape 16 de remplissage de chaque enceinte de l'ossature du fantôme associé à un organe radiologique est remplie par un gel dosimétrique dont les caractéristiques physiques sont proches de la classe des compositions chimiques et des masses volumiques de l'organe radiologique.

De manière préférée, le remplissage du fantôme est mis en oeuvre en phase liquide pour chacun des gels, et les gels sont laissés au repos dans les cavités pour gélifier ensuite à l'état de gel proprement dit, le temps de gélification étant fonction de la masse totale de gel contenu dans le fantôme.

Le remplissage des volumes internes des enceintes est assuré au moyen des tuyaux reliant les enceintes à l'extérieur du fantôme à vide.

Lors de l'étape de remplissage 16, un ou plusieurs gels dosimétriques, par exemple un gel de Fricke ou un gel polymère sont utilisés. De préférence, les caractéristiques de ces gels sont proches des compositions chimiques et des masses volumiques des différents organes qu'ils simulent.

Suivant la Figure 2, un procédé 102 de détermination expérimentale d'une distribution volumique de doses de rayonnement reçues par un fantôme, représentative d'une distribution de doses qui seraient reçues par un organisme biologique placé dans de mêmes conditions d'irradiation prédéterminées, comprend une première étape 104 de fourniture d'un fantôme physique radiométrique, fabriqué selon le procédé de production 2 de la Figure 1 ou l'une ou plusieurs de ses variantes.

Le procédé de détermination expérimentale 102 comprend en outre des deuxième, troisième étapes 106 et 108, exécutées successivement après l'étape de fourniture 104 du fantôme physique.

Dans la deuxième étape 106, le fantôme physique fourni dans la première étape de fourniture 102 est irradié sous les conditions d'irradiation prédéterminées qui ont régi la conception et la réalisation du fantôme physique fourni, et qui sont identiques à celles auxquelles un patient serait ou aurait été soumis, par exemple auprès de moyens d'irradiation à visée thérapeutique en radiothérapie comme par exemple un accélérateur linéaire médical (LINAC) ou auprès de toutes installations d'irradiation à visée diagnostic ou encore auprès d'installation reproduisant les conditions lors de la survenue d'un accident d'irradiation.

Les rayonnements utilisés sont des rayonnements ionisants par exemple de photons, électrons, protons, et noyaux lourds.

Puis dans la troisième étape 108, il est procédé à une lecture 110 d'un paramètre physique des gels irradiés in situ dans le fantôme physique en plaçant le fantôme physique rempli des gels irradiés dans un appareil d'imagerie, et à l'encodage 112 des lectures du paramètre physique sous la forme de données brutes de lecture. L'appareil d'imagerie est un appareil utilisant par exemple un procédé par transmission optique ou un appareil d'imagerie à Résonance Magnétique Nucléaire RMN.

Les informations brutes de lecture représentent un paramètre physique lu, par exemple une densité optique, un temps de relaxation.

Ensuite, dans la même troisième étape 108, les données brutes de lecture sont traitées 114 pour déterminer une image de la distribution tridimensionnelle des doses de rayonnement reçues dans la partie ou la totalité de l'organisme biologique.

L'étape de traitement des données de lecture 114 comprend les sous-étapes 116, 118, consistant à traduire 116 à partir d'un étalonnage dosimétrique des gels l'information brute de lecture en termes de doses absorbées suivant une distribution tridimensionnelle, et à regrouper les données traduites 118 en termes de doses absorbées pour créer des images particulières comprises dans l'ensemble des coupes bidimensionnelles selon un plan de coupe prédéterminé, des surfaces « isodose en trois dimensions, des images proposées par les logiciels de planification de traitement de radiothérapie.

Cette distribution des doses est comparée à celle fournie par le système de planification du traitement de radiothérapie ou tout autre moyen de détermination de la distribution en 3D ou de reconstitution informatique de l'accident. La comparaison des deux distributions permet selon le cas, une validation des modalités du traitement avant d'avoir réellement irradié le patient, ou de vérifier les circonstances de l'accident et de donner accès à des informations utiles pour le diagnostic et le traitement.

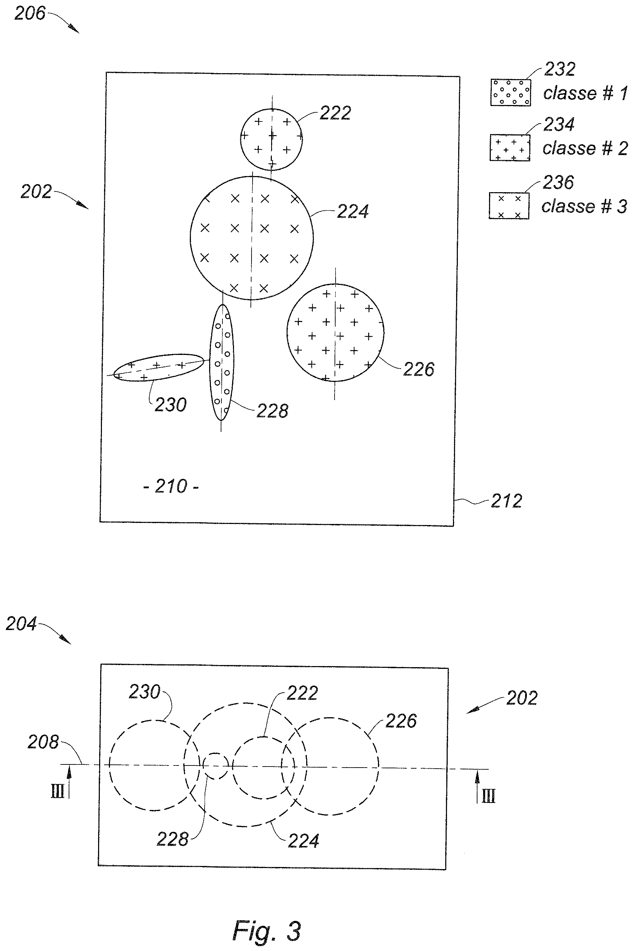

Suivant la Figure 3, un modèle radiologique 202 schématique et purement illustratif est représenté sous la forme d'une première vue globale de dessus 204 et d'une deuxième vue 206 d'une coupe du modèle radiologique 202 suivant un plan médian 208 de coupe III-III perpendiculaire à la vue de dessus 204.

Le modèle radiologique comprend un milieu 210 correspondant à un gel de caractéristiques équivalentes en moyenne à celles des organes non identifiés contenus dans un parallélépipède 212, des organes radiologiques 222, 224, 226, 228, 230, ici au nombre de cinq, étant identifiés dans ce même parallélépipède 212.

Les volumes des organes radiologiques 222, 224, 226 sont ici de manière simplifiée des sphères de rayons différents, tandis que les volumes des organes radiologiques 228 et 230 sont respectivement un cylindre allongé selon un axe d'orientation verticale sur la vue de coupe 206, et un disque aplati dans un plan perpendiculaire au plan de coupe 208, le cylindre et le disque ayant des formes adoucies.

L'organe radiologique 228 est constitué d'une première classe de matériau radiologique correspondant à un premier motif de hachurage 232 tandis que les organes radiologiques 222, 230 sont constitués d'une deuxième classe de matériau radiologique correspondant à un deuxième motif de hachurage 234.

Les organes radiologiques 222, 226, 230 sont constitués d'une troisième classe de matériau radiologique correspondant à un troisième motif de hachurage 236.

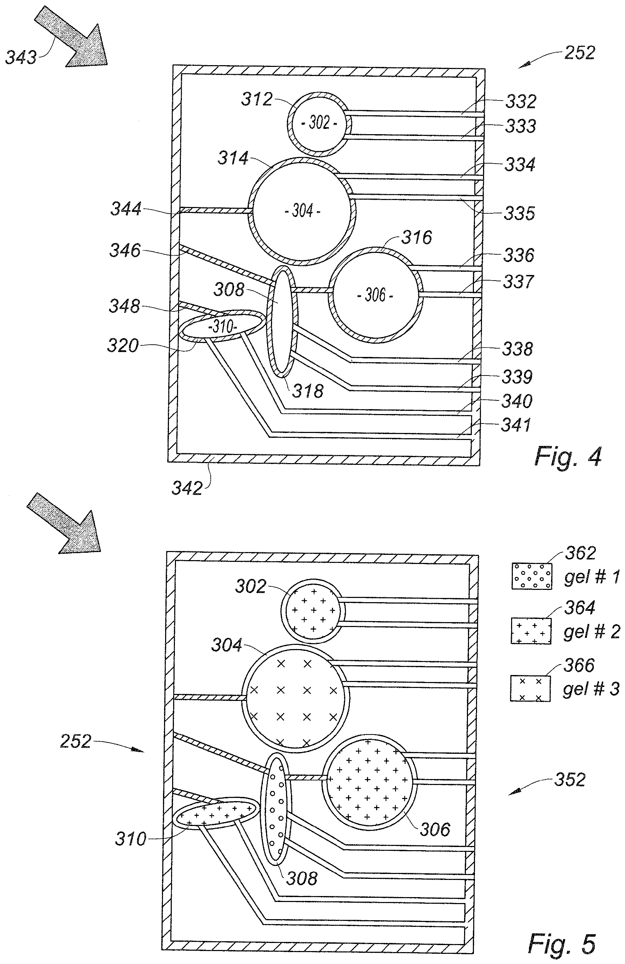

La Figure 4 est une vue d'une coupe d'une ossature 252 d'un fantôme physique obtenue après l'impression 3D. L'ossature 252 du fantôme physique a été obtenue à partir du modèle radiologique 202 de la Figure 3 en mettant en oeuvre les étapes de la Figure 1.

L'ossature 252 du fantôme physique comprend des enceintes vides 302, 304, 306, 308, 310, définies respectivement par les volumes des organes radiologiques de la Figure 3, et délimités respectivement par des parois 312, 314, 316, 318, 320 constituées d'une ou de plusieurs encres d'impression 3D durcissables, transparentes au rayonnement si leur épaisseur est suffisamment fine ou ayant des caractéristiques d'interaction avec le rayonnement ionisant similaires à celles des tissus simulés ou à celles des gels qu'elles confinent.

Chaque enceinte 302 ; 304 ; 306 ; 308 ; 310 est ici pourvue sur sa paroi d'un orifice de remplissage d'entrée du gel et d'un office de remplissage d'évacuation de l'air, raccordés respectivement à des conduits de remplissage 332, 333 ; 334, 335 ; 336, 337 ; 338, 339 ; 340, 341 débouchant à l'extérieur de l'ossature 252 et raccordés à une structure de maintien 342 en position des enceinte entre elles.

Les emplacements et les diamètres des orifices de remplissage, les diamètres et les trajets de conduits de remplissage 332, 333 ; 334, 335 ; 336, 337 ; 338, 339 ; 340, 341 sont configurés pour permettre le remplissage de chaque enceinte 302 ; 304 ; 306 ; 308 ; 310 par son gel associé sans formation de bulle de volume.

Les emplacements et les diamètres des orifices de remplissages, les diamètres et les trajets de conduits de remplissage 332, 333 ; 334, 335 ; 336, 337 ; 338, 339 ; 340, 341 sont également configurés pour éviter que les conduits ne perturbent la distribution attendue des doses dans la zone du fantôme correspondant à la zone à traiter de l'organisme biologique en fonction de l'emplacement de la zone à traiter et de l'angle d'entrée du faisceau de rayonnement. Ici, la zone à traiter est supposée située sur la partie supérieure gauche de la Figure 4 du fantôme avant remplissage des gels 252 et le faisceau du rayonnement est dirigée suivant la flèche 343. Les orifices et les conduits de remplissage sont en conséquence disposés ici sur le côté droit de la Figure 4. Ici, le routage des conduits de remplissage est simple mais il peut être beaucoup plus complexe dans d'autres cas.

Il est remarquer qu'ici le système de remplissage, formé par les conduits de remplissage contribue au maintien des enceintes entre elles en complément de l'action d'autres structures de maintien 344, 346, 348.

En variante, le système de remplissage et le système de maintien sont séparés.

Suivant la Figure 5, un fantôme physique 352 rempli des gels dosimétriques non irradiés, obtenu à la fin du procédé de production de la Figure 1 comporte l'ossature 252 du fantôme de la Figure 4 dans laquelle les enceintes 302, 304, 306, 308, 310 sont remplies de leurs gels dosimétriques dont les masses volumique et les compositions chimiques correspondent aux classes des organes radiologiques du modèle radiologique de la Figure 3.

Ici, l'enceinte 308 est remplie d'un premier gel 362 correspondant à la première classe radiologique, les enceintes 302, 306, 310 sont remplies d'un deuxième gel 364 correspondant à la deuxième classe radiologique, et l'enceinte 304 est remplie d'un troisième gel 366 correspondant à la troisième classe radiologique.

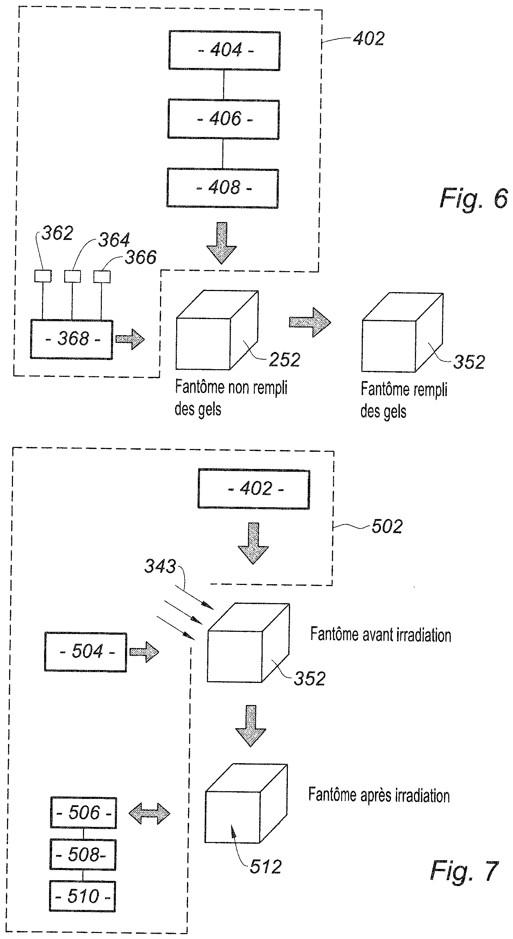

Suivant la Figure 6, un système de production 402 du fantôme physique radiométrique 352 de la Figure 5 mettant en oeuvre le procédé de production 2 du fantôme de la Figure 1 est illustré.

Le système de production 402, délimité par une bordure en traits pointillés sur la Figure 6, comprend un moyen de fourniture 404 d'une ou de plusieurs images tridimensionnelles anatomiques des tissus de l'organisme biologique en termes de compositions chimiques et de masses volumiques, un calculateur électronique 406, une l'imprimante 3D 408, au moins deux gels dosimétriques différents, ici les trois gels 362, 364, 366, et un moyen de remplissage 368 du fantôme vide 352 par les gels dosimétriques 362, 364, 366.

Le calculateur électronique 406 est configuré pour déterminer un modèle tridimensionnel 3D radiologique représentatif de l'absorption et la diffusion du rayonnement au sein de l'organisme biologique, en regroupant dans des organes radiologiques les tissus qui sont adjacents entre eux et qui ont des compositions chimiques et des masses volumiques voisines. Chaque organe radiologique occupe un volume d'organe radiologique, délimité par une surface d'enveloppe correspondante. Le volume radiologique est égal ou approche par lissage de ses contours la somme des volumes des tissus regroupés au sein de l'organe radiologique. Chaque organe radiologique est caractérisé par une classe de compositions chimiques et de masses volumiques voisines.

Le calculateur électronique 406 est également configuré pour, à partir du modèle tridimensionnel radiologique, déterminer suivant l'étape 8 de la Figure 1 les plans d'une ossature matérielle de fantôme physique comme étant l'ensemble des enveloppes des organes radiologiques ou des parois d'enceintes d'organe, maintenues en position relative, l'ensemble des conduits de remplissage, l'ensemble des supports de maintien, et traduire les plans de l'ossature en des commandes de sa réalisation par une imprimante 3D prédéterminée.

L'imprimante 3D 408 est configurée pour réaliser l'ossature matérielle 252 du fantôme non rempli des gels dosimétriques, à l'aide des encres d'impression dont les propriétés d'interaction avec les rayonnements ionisants sont voisines de celles des gels et des tissus biologiques qu'ils représentent.

Les imprimantes 3D actuellement commercialisées permettent l'impression d'un volume de 60 x 60 x 60 cm3, un tel volume étant suffisant pour représenter toutes parties d'un patient. Les parois des volumes radiologiques représentant les organes peuvent être en plastique sans apporter de perturbation notable à la mesure mais des compositions proches de celles des tissus peuvent être utilisées lorsqu'elles existent.

Les gels dosimétriques sont destinés à remplir chacun une enceinte radiologique différente, associée à un organe radiologique différent en termes de classe de compositions chimiques et de masses volumiques. Les gels dosimétriques sont des substances de texture gélatineuse à la température ambiante qui contiennent une espèce dont la nature est modifiée lors de l'irradiation, par exemple du fer ferreux qui est transformé en du fer ferrique pour un gel de Fricke, ou un phénomène de polymérisation pour un gel polymère. De tels gels sont décrits par exemple dans l'article de L.J. Scheiner, intitulé « Review of Fricke gel dosimeters », publié dans Journal of Physics : Conférence Seires3 (2004) 9-21.

Suivant la Figure 7, un système de détermination expérimentale 502 d'une distribution volumique de doses de rayonnement reçues par le fantôme de la Figure 5 produit par le système de production de la Figure 6 et mettant en oeuvre le procédé 102 de détermination expérimentale de la Figure 2, comprend un système de production 402 d'un fantôme physique radiométrique rempli de gels dosimétriques tel que décrit dans la Figure 6, un moyen d'irradiation 504 du fantôme physique par un rayonnement sous des conditions d'irradiation prédéterminées, un moyen de lecture 506 d'un paramètre physique des gels in situ dans le fantôme, un moyen d'encodage du paramètre physique lu 508 sous forme de données brutes de lecture, et un moyen de traitement 510 des données brutes de lecture.

Le moyen de lecture 506 est mis en oeuvre en plaçant le fantôme physique, rempli des gels irradiés 512, dans un appareil d'imagerie utilisant par exemple un procédé par transmission optique ou dans un appareil d'imagerie RMN.

Le moyen de traitement 510 des données brutes de lecture est configuré pour déterminer une image de la distribution tridimensionnelle des doses de rayonnement reçues dans la partie ou la totalité de l'organisme biologique.

Deux exemples d'applications de l'invention sont décrits ci-dessous.

Selon un premier exemple concernant la radiothérapie, lors de la planification d'un traitement, une série d'images diagnostic est réalisée au moyen d'une technique classique telle que le scanner ou la RMN. Ces images sont utilisées par l'équipe médicale pour délimiter les contours de la tumeurs et ensuite introduite dans des programmes de planification des traitements qui génèrent un plan de traitement définissant la qualité, la collimation des faisceaux, le nombre et l'incidence des faisceaux de rayonnement sur le patient. Dans ce cas, la réalisation du traitement, non pas sur le patient mais sur le fantôme physique qui le représente avec précision, permet de comparer la distribution des doses telle que planifiée et celle réellement administrée au fantôme. C'est là un outil de validation a priori du traitement. Il est également possible a posteriori de reproduire pour l'étudier un traitement qui a déjà été administré. Dans les deux cas, la mesure donne accès à des informations sur la distribution des doses en dehors des volumes généralement explorés par les logiciels de planification. De telles données peuvent s'avérer très intéressantes dans le cas des études sur les seconds cancers qui pourraient être radio-induits.

Selon un deuxième exemple concernant la reconstitution d'un accident radiologique, suite à un accident radiologique, le traitement des personnes irradiées dépend de la façon dont ils ont été irradiés, c'est-à-dire de la distribution des doses dans les différents organes. Cette distribution des doses peut être calculée au moyen de logiciel de reconstitution d'accident, voire estimée à partir de mesure dans des fantômes standard. La mesure de cette distribution des doses dans un fantôme anthropomorphique réaliste, représentatif de la "géométrie" réelle interne et externe du patient est alors comparée à celle calculée. Cette comparaison permet de valider les résultats des calculs, par conséquent de vérifier les circonstances de l'accident, et de donner accès à des informations utiles pour le diagnostic et le traitement.

Dans ces deux exemples, l'utilisation de fantômes anthropomorphiques tels que proposés par l'invention et représentatifs du patient dans son particularisme, permet, associée à un moyen de mesure qui évalue en trois dimensions et en une seule mesure la distribution des doses dans le patient, par exemple un scanner ou une station RMN, permet une validation plus précise et plus rapide

Les fantômes de l'invention seront le plus souvent des fantômes anthropomorphiques et pourront être utilisés pour tout type de radiothérapie externe : RCMI/IMRT (et toutes irradiations rotationnelles - Tomothérapie, VMAT, Rapidarc ...) ; stéréotaxie ; thérapie proton/hadron; curiethérapie.

Cette utilisation s'inscrit dans le processus d'assurance qualité (vérification expérimentale des traitements) ou encore d'étude à posteriori des risques de « second cancer ». En fait plus généralement, pour inclure les cas de reconstitution d'accident, ces fantômes sont utiles dans tous les domaines ou une connaissance de la distribution des doses dans le patient est nécessaire a priori ou a posteriori : test et validation des traitements en radiothérapie, test et validation d'exposition aux rayonnements ionisants à visée diagnostic, étude a posteriori de traitement déjà prodigués, et reconstitution d'accident radiologique.

## Revendications

1. Procédé de production d'un fantôme physique radiométrique d'un organisme biologique pour déterminer expérimentalement sous des conditions d'irradiation prédéterminées une distribution volumique des doses de rayonnement reçues par le fantôme, représentative de la distribution des doses qui seraient reçues par l'organisme biologique placée dans les mêmes conditions d'irradiation, l'organisme biologique ayant au moins deux volumes de tissus biologiques dont les compositions chimiques et les masses volumiques sont nettement différentes,
le procédé de production comprenant les étapes consistant à :
à partir d'une ou de plusieurs images tridimensionnelles anatomiques des tissus en termes de compositions chimiques et de masses volumiques, déterminer (6) un modèle tridimensionnel radiologique, représentatif de l'absorption et la diffusion du rayonnement à l'intérieur de l'organisme biologique, en regroupant dans des organes radiologiques les tissus adjacents entre eux et ayant des compositions chimiques et des masses volumiques voisines,
chaque organe radiologique occupant géométriquement dans l'espace un volume radiologique, égal ou approchant par lissage des contours de son enveloppe la somme des volumes de tous les tissus regroupés dans le même organe radiologique, et étant **caractérisé par** une classe radiologique identifiant la plage des compositions chimiques et des masses volumiques voisines des tissus regroupés dans le même organe radiologique ; puis
à partir du modèle radiologique, réaliser (10) à l'aide d'une impression 3D une ossature matérielle du fantôme physique en reproduisant chaque volume radiologique par une enceinte remplie d'air et délimitée par une paroi, la paroi étant constituée d'une encre d'impression 3D; puis
remplir (16) chaque enceinte de l'ossature matérielle du fantôme, associée à un organe radiologique par un gel dosimétrique dont les caractéristiques physiques sont proches ou identiques à celles de la classe des compositions chimiques et des masses volumiques de l'organe radiologique.

2. Procédé de production d'un fantôme physique radiométrique selon la revendication 1, dans lequel, pendant la détermination (6) du modèle radiologique,
pour chaque regroupement de tissus biologiques dans un même organe radiologique,
un volume radiologique intermédiaire est d'abord déterminé comme étant égal à la somme des volumes des tissus regroupés dans l'organe radiologique, puis
le volume radiologique de l'organe radiologique est déterminé en lissant les contours du volume radiologique intermédiaire afin de limiter les anfractuosités et faciliter le remplissage du gel dosimétriqe.

3. Procédé de production d'un fantôme physique radiométrique selon la revendication 2, comprenant en outre une étape (8) de préparation de plans logiciels de fabrication de l'ossature matérielle du fantôme et de traduction de ces plans en un programme de commandes d'impression 3D de l'ossature, insérée entre l'étape (6) de détermination du modèle radiologique et l'étape (10) d'impression 3D de l'ossature du fantôme, dans laquelle étape de préparation (8),
pour chaque enceinte associée à un organe radiologique, le nombre des orifices de remplissage d'entrée de gel, le nombre des orifices de remplissage de sortie d'air, les emplacements et les diamètres du ou des orifices de remplissage d'entrée de gel et du ou des orifices de remplissage de sortie d'air, ainsi que les trajets et les diamètres des conduits de remplissage de l'enceinte, reliés entre les orifices de remplissage et l'extérieur du fantôme, sont déterminés de sorte
à permettre le remplissage de l'organe radiologique sans bulles de volume.

4. Procédé de production d'un fantôme physique radiométrique selon la revendication 3 dans lequel le nombre total des orifices de remplissage, les emplacements des orifices de remplissage et les trajets des conduits de remplissage de l'ensemble des enceintes sont déterminés pour ne pas gêner la propagation du rayonnement vers une zone à traiter en fonction de l'emplacement de la zone à traiter et/ou de l'angle d'entrée du faisceau de rayonnement.

5. Procédé de production d'un fantôme radiométrique selon la revendication 4 dans les formes lissées des volumes des enceintes sont également déterminées pour ne pas gêner la propagation du rayonnement vers une zone à traiter en fonction de l'emplacement de la zone à traiter et/ou de l'angle d'entrée du faisceau de rayonnement.

6. Procédé de production d'un fantôme radiométrique selon l'une quelconque des revendications 1 à 5 dans lequel les gels dosimétriques sont compris dans l'ensemble formé par les gels de Fricke et les gels polymère.

7. Procédé de détermination expérimentale d'une distribution volumique de doses de rayonnement reçues par un fantôme physique radiométrique représentative d'une distribution de doses qui seraient reçues par la partie ou de la totalité d'un organisme biologique placé dans de mêmes conditions d'irradiation prédéterminées, comprenant les étapes consistant à :
Fournir (104) un fantôme physique rempli de ses gels dosimétriques et fabriqué selon l'une quelconque des revendications 1 à 6 ;
Irradier (106) ensuite le fantôme physique sous les conditions d'irradiation prédéterminées ;
Procéder (108) à une lecture (110) d'un paramètre physique des gels in situ dans le fantôme en plaçant le fantôme rempli des gels irradiés dans un appareil d'imagerie utilisant par exemple un procédé par transmission optique ou dans un appareil d'imagerie RMN et l'encoder (112) sous forme de données brutes de lecture ;
Traiter (114) ensuite les données brutes de lecture pour déterminer une image de la distribution tridimensionnelle des doses de rayonnement reçues dans la partie ou la totalité de l'organisme biologique.

8. Procédé de détermination expérimentale d'une distribution volumique de doses de rayonnement reçues par un fantôme physique selon la revendication 7, **caractérisé en ce que** les informations brutes de lecture représentent un paramètre physique lu compris dans l'ensemble formé par une densité optique, un temps de relaxation.

9. Procédé de détermination expérimentale d'une distribution volumique de doses de rayonnement reçues par un fantôme physique selon l'une des revendications 7 et 8, dans laquelle l'étape de traitement des données de lecture (114) comprend les étapes consistant à
Traduire (116) l'information brute de lecture en termes de doses absorbées ; et
Regrouper (118) les données traduites en termes de doses absorbées pour créer des images particulières comprises dans l'ensemble des coupes bidimensionnelles selon un plan de coupe prédéterminé, des surfaces « isodose » en trois dimensions, des images proposées par les logiciels de planification de traitement de radiothérapie.

10. Procédé de détermination expérimentale d'une distribution volumique de doses dues aux rayonnement reçues par un fantôme selon l'une des revendications 7 à 9, dans lequel les rayonnements sont des rayonnements de particules comprises dans l'ensemble formé par les photons, les électrons, les protons, les noyaux lourds.

11. Fantôme physique radiométrique d'un organisme biologique pour déterminer expérimentalement sous des conditions d'irradiation prédéterminées une distribution volumique des doses de rayonnement reçues par le fantôme représentative de la distribution des doses qui seraient reçues par l'organisme biologique placée dans les mêmes conditions d'irradiation, l'organisme biologique ayant au moins deux volumes de tissus biologiques dont les compositions chimiques et les masses volumiques sont nettement différentes, **caractérisé en ce que** le fantôme physique comprend
au moins deux enceintes (302, 304,308), maintenues en position dans un cadre de support (342), délimitées par des parois respectives constituées d'encre solide d'impression 3D transparente au rayonnement, et remplie chacune d'un gel dosimétrique différent (364, 366, 362),
les au moins deux enceintes (302, 304, 308) ayant des volumes radiologiques différents et séparés, chaque volume radiologique étant identique ou s'approchant par lissage des contours de la somme des volumes d'un ensemble complet de tissus biologiques regroupés de l'organisme biologique imité radiologiquement et déterminés à partir d'une ou de plusieurs images tridimensionnelles anatomiques des tissus en termes de compositions chimiques et de masses volumiques, lesdits tissus regroupés étant adjacents entre eux et ayant des compositions chimiques et des masses volumiques voisines, et
les gels dosimétriques (364, 366, 362), remplissant lesdites enceintes (302, 304, 308) ayant chacun des caractéristiques physiques proches des compositions chimiques et des masses volumiques des tissus biologiques adjacents regroupés dont la somme des volumes est identique ou est approchée par lissage du volume radiologique de l'enceinte que le gel remplit.

12. Fantôme radiométrique selon la revendication 11 dans lequel
chaque enceinte (302 ; 304 ; 308) comporte au moins un orifice de remplissage d'entrée de gel et au moins un orifice de remplissage de sortie d'air, et au moins deux conduits de remplissage (332,333 ; 334, 335 ;338, 339) reliés entre les orifices de remplissage et l'extérieur du fantôme, et
pour chaque enceinte (302 ; 304 ; 308), le nombre des orifices de remplissage d'entrée de gel, le nombre des orifices de remplissage de sortie d'air, les emplacements et les diamètres du ou des orifices de remplissage d'entrée de gel et du ou des orifices de remplissage de sortie d'air, ainsi que les trajets et les diamètres des conduits de remplissage de l'enceinte, reliés entre les orifices de remplissage et l'extérieur du fantôme, sont configurés pour permettre le remplissage de l'enceinte à un débit prédéterminé sans création de bulle de volume.

13. Système de production d'un fantôme physique radiométrique simulant les propriétés d'absorption ou de diffusion d'un rayonnement d'un organisme biologique, l'organisme biologique ayant au moins deux volumes de tissus biologiques dont les compositions chimiques et les masses volumiques sont nettement différentes,
le système comprenant
un moyen de fourniture (404) d'une ou de plusieurs images tridimensionnelles anatomiques des tissus en termes de compositions chimiques et de masses volumiques,
un calculateur électronique (406) configuré pour
à partir d'une ou de plusieurs images tridimensionnelles anatomiques des tissus en termes de compositions chimiques et de masses volumiques, déterminer (6) un modèle tridimensionnel radiologique, représentatif de l'absorption et la diffusion du rayonnement à l'intérieur de l'organisme biologique, en regroupant dans des organes radiologiques les tissus adjacents entre eux et ayant des compositions chimiques et des masses volumiques voisines,
chaque organe radiologique occupant géométriquement dans l'espace un volume radiologique, égal ou approchant par lissage des contours de son enveloppe la somme des volumes de tous les tissus regroupés dans le même organe radiologique, et étant **caractérisé par** une classe radiologique identifiant la plage des compositions chimiques et des masses volumiques voisines des tissus regroupés dans le même organe radiologique, et
à partir du modèle tridimensionnel radiologique déterminer les plans d'une ossature matérielle du fantôme comme étant un ensemble d'enceintes, de conduits de remplissage en gel des enceintes, et d'éléments de support pour maintenir en position les enceintes entre elles, puis traduire les plans de l'ossature matérielle en un programme de commandes d'impression 3D à exécuter par une imprimante 3D prédéterminée (408),
l'imprimante 3D prédéterminée (408) configurée pour réaliser l'ossature matérielle du fantôme non rempli des gels dosimétriques, à l'aide d'encres d'impression 3D transparentes au rayonnement ;
au moins deux gels dosimétriques différents (362, 364, 366) destinés à remplir chacun une enceinte radiologique différente associée à un organe radiologique différent en termes de classe de compositions chimiques et de masses volumiques, et
un moyen de remplissage (410) du fantôme vide par les aux moins deux gels dosimétriques.

14. Système de production d'un fantôme physique radiométrique selon la revendication 13, dans lequel le calculateur électronique (406) est configuré pour chaque regroupement de tissus biologiques dans un organe radiologique,
Déterminer un volume radiologique intermédiaire comme étant égal à la somme des volumes des tissus regroupés dans l'organe radiologique, et
Déterminer le volume radiologique de l'organe radiologique en lissant les contours du volume radiologique intermédiaire afin de limiter les anfractuosités et faciliter le remplissage futur du gel.

15. Système de production d'un fantôme physique radiométrique selon l'une quelconque des revendications 13 à 14, dans lequel le calculateur électronique (406) est configuré pour chaque organe radiologique,
Déterminer le nombre des orifices de remplissage d'entrée de gel, le nombre des orifices de remplissage de sortie d'air, chaque emplacement et diamètre du ou des orifices de remplissage d'entrée de gel, chaque emplacement et diamètre du ou des orifices de remplissage de sortie d'air, ainsi que les trajets et les diamètres des conduits de remplissage des volumes des organes radiologiques, reliés entre les orifices de remplissage et l'extérieur du fantôme, de sorte
à permettre le remplissage de l'organe radiologique sans bulles de volume.

16. Système de production d'un fantôme physique radiométrique selon la revendication 15, dans lequel le calculateur électronique (406) est configuré pour déterminer le nombre total des orifices de remplissage, les emplacements des orifices de remplissage et les trajets des conduits de remplissage de sorte à ce que les conduits ne gênent pas la propagation du rayonnement vers une zone à traiter en fonction de l'emplacement de la zone à traiter, de l'angle d'entrée du faisceau de rayonnement.

17. Système de production d'un fantôme physique radiométrique selon la revendication 16, dans lequel le calculateur électronique (406) est configuré pour déterminer également les formes lissés des volumes des organes radiologiques pour ne pas modifier significativement la propagation du rayonnement vers une zone à traiter en fonction de l'emplacement de la zone à traiter, de l'angle d'entrée du faisceau de rayonnement.

18. Système de détermination expérimentale d'une distribution volumique de doses de rayonnement reçues par un fantôme représentative d'une distribution de doses qui seraient reçues par la partie ou de la totalité d'un organisme biologique placé dans de mêmes conditions d'irradiation prédéterminées, comprenant :
Un système de production (402) d'un fantôme physique radiométrique rempli de gels dosimétriques défini selon l'une quelconque des revendications 13 à 17 ;
Un moyen d'irradiation (504) du fantôme physique (352) par un rayonnement sous des conditions d'irradiation prédéterminées ;
Un moyen de lecture (506) d'un paramètre physique des gels in situ dans le fantôme lorsque ce dernier rempli des gels irradiés est placé dans un appareil d'imagerie utilisant par exemple un procédé par transmission optique ou dans un appareil d'imagerie RMN et un moyen d'encodage du paramètre physique lu sous forme de données brutes de lecture ;
Un moyen de traitement (508) des données brutes de lecture pour déterminer une image de la distribution tridimensionnelle des doses de rayonnement reçues dans l'organisme biologique.

19. Produit programme d'ordinateur comportant un ensemble d'instructions qui lorsqu'elles sont chargées et exécutés par le calculateur électronique (406) et/ou le moyen de traitement (508) des données brutes de lecture du système de production (402) et du système de détermination expérimentale (502) définies respectivement dans les revendications mettent en oeuvre une partie des étapes du procédé de production défini selon les revendications 1 à 6 et dans le procédé de détermination expérimentale défini selon les revendications 7 à 10, en particulier les étapes (6), (8), (106) et (108).

## Patentansprüche

1. Verfahren zur Herstellung eines radiometrischen physischen Phantoms eines biologischen Organismus zum experimentellen Bestimmen, unter vorher festgelegten Bestrahlungsbedingungen, einer Volumenverteilung der durch das Phantom empfangenen Strahlungsdosen, die für die Verteilung der Dosen repräsentativ ist, die von dem biologischen Organismus, unter den gleichen Bestrahlungsbedingungen platziert, empfangen würden, wobei der biologische Organismus mindestens zwei biologische Gewebevolumina aufweist, von denen die chemischen Zusammensetzungen und die Dichten deutlich verschieden sind,
wobei das Verfahren zur Herstellung die Schritte umfasst, bestehend aus:
ausgehend von einem oder mehreren dreidimensionalen anatomischen Bildern der Gewebe, hinsichtlich der chemischen Zusammensetzungen und Dichten, dem Bestimmen (6) eines dreidimensionalen radiologischen Modells, das repräsentativ ist für die Absorption und die Diffusion der Strahlung im Inneren des biologischen Organismus, durch Zusammenfassen der untereinander angrenzenden, und benachbarte chemische Zusammensetzungen und Dichten aufweisenden, Gewebe in radiologischen Organen,
wobei jedes radiologische Organ im Raum geometrisch ein gleiches, oder durch Glätten der Konturen seiner Hülle der Summe der Volumina aller zusammengefassten Gewebe in dem gleichen radiologischen Organ annäherndes radiologisches Volumen einnimmt, und **gekennzeichnet durch** eine radiologische Klasse, die den von den in dem gleichen radiologischen Organ zusammengefassten Geweben benachbarten Bereich chemischer Zusammensetzungen und Dichten identifiziert; danach
ausgehend von dem radiologischen Modell, Fertigen (10) eines materiellen Rahmenwerks des physischen Phantoms mithilfe eines 3D-Drucks, indem jedes radiologische Volumen durch ein mit Luft gefülltes und durch eine Wand umschlossenes Entladungsgefäß reproduziert wird, wobei die Wand aus einer Tinte zum 3D-Druck besteht; danach
Füllen (16) jedes mit einem radiologischen Organ assoziierten Entladungsgefäßes des materiellen Rahmenwerks des Phantoms durch ein Dosimetrie-Gel, dessen physischen Eigenschaften nahe an oder identisch zu denen der Klasse von chemischen Zusammensetzungen und Dichten des radiologischen Organs sind.

2. Verfahren zur Herstellung eines radiometrischen physischen Phantoms nach Anspruch 1, wobei, während der Bestimmung (6) des radiologischen Modells,
für jede Zusammenfassung von biologischen Geweben in einem gleichen radiologischen Organ,
erst ein zwischenliegendes radiologisches Volumen als gleich der Summe der in dem radiologischen Organ zusammengefassten Gewebevolumina bestimmt wird, danach
das radiologische Volumen des radiologischen Organs durch Glätten der Konturen des zwischenliegenden radiologischen Volumens bestimmt wird, um die Einbuchtungen einzugrenzen und das Einfüllen des Dosimetrie-Gels zu erleichtern.

3. Verfahren zur Herstellung eines radiometrischen physischen Phantoms nach Anspruch 2, weiter einen Schritt (8) der Vorbereitung von Softwareplänen zur Anfertigung des materiellen Rahmenwerks des Phantoms und des Überführens dieser Pläne in ein Steuerungsprogramm des 3D-Drucks des Rahmenwerks umfassend, eingefügt zwischen dem Schritt (6) des Bestimmens des radiologischen Modells und dem Schritt (10) des Druckens des Rahmenwerks des Phantoms, wobei in dem Schritt (8) der Vorbereitung
(für jedes mit einem radiologischen Organ assoziierten Entladungsgefäß, die Anzahl von Befüllungsöffnungen zum Geleinlass, die Anzahl von Befüllungsöffnungen zum Luftauslass, die Lagen und die Durchmesser der Befüllungsöffnung(en) zum Geleinlass und der Befüllungsöffnung(en) zum Luftauslass, sowie die Strecken und die Durchmesser der Befüllungsleitungen des Entladungsgefäßes, die zwischen den Befüllungsöffnungen und dem Äußeren des Phantoms verbunden sind, auf eine Art und Weise bestimmt werden,
um das Befüllen des radiologischen Organs ohne Volumenblasen zu erlauben.

4. Verfahren zur Herstellung eines radiometrischen physischen Phantoms nach Anspruch 3, wobei die Gesamtanzahl an Befüllungsöffnungen, die Lagen der Befüllungsöffnungen und die Strecken der Befüllungsleitungen aller Entladungsgefäße bestimmt werden, um die Ausbreitung der Strahlung zu einer zu behandelnden Zone in Abhängigkeit von der Lage der zu behandelnden Zone und/oder des Eintrittswinkels des Strahlenbündels nicht zu stören.

5. Verfahren zur Herstellung eines radiometrischen Phantoms nach Anspruch 4, wobei die geglätteten Formen der Volumina der Entladungsgefäße ebenfalls bestimmt werden, um die Ausbreitung der Strahlung zu einer zu behandelnden Zone in Abhängigkeit von der Lage der zu behandelnden Zone und/oder des Eintrittswinkels des Strahlenbündels nicht zu stören.

6. Verfahren zur Herstellung eines radiometrischen Phantoms nach einem der Ansprüche 1 bis 5, wobei die Dosimetrie-Gele in der Einheit umfasst sind, die durch Fricke-Gele und Polymer-Gele gebildet werden.

7. Verfahren zum experimentellen Bestimmen einer Volumenverteilung der durch das radiometrische physische Phantom empfangenen Strahlungsdosen, repräsentativ für eine Dosisverteilung, die durch den Teil oder die Gesamtheit eines unter den gleichen vorher festgelegten Bestrahlungsbedingungen platzierten biologischen Organismus empfangen würde, umfassend die Schritte bestehend aus:
Bereitstellen (104) eines mit seinen Dosimetrie-Gelen gefüllten und nach einem der Ansprüche 1 bis 6 angefertigten physischen Phantoms;
daraufhin Bestrahlen (106) des physischen Phantoms unter den vorher festgelegten Bestrahlungsbedingungen;
Vornehmen (108) eines Auslesens (110) eines physischen Parameters der Gele in-situ in dem Phantom, indem das mit den bestrahlten Gelen befüllte Phantom in ein Bildgebungsgerät platziert wird, das zum Beispiel ein Verfahren durch optische Übertragung nutzt, oder in ein NMR-Bildgebungsgerät und Kodieren (112) derselben in Form von Ausleserohdaten ;
daraufhin Behandeln (114) der Ausleserohdaten zum Bestimmen eines Bilds der dreidimensionalen Verteilung von in dem Teil oder der Gesamtheit des biologischen Organismus empfangenen Strahlungsdosen.

8. Verfahren zum experimentellen Bestimmen einer Volumenverteilung von durch ein physisches Phantom nach Anspruch 7 empfangenen Strahlungsdosen, **dadurch gekennzeichnet, dass** die Ausleserohinformationen einen ausgelesenen physischen Parameter darstellen, der in der durch eine optische Dichte, eine Relaxationszeit gebildeten Einheit umfasst ist.

9. Verfahren zum experimentellen Bestimmen einer Volumenverteilung von durch ein physisches Phantom nach einem der Ansprüche 7 und 8 empfangenen Strahlungsdosen, wobei der Behandlungsschritt der Auslesedaten (114) die Schritte umfasst, bestehend aus
Überführen (116) der Ausleserohdaten hinsichtlich der absorbierten Dosen; und
Zusammenfassen (118) der überführten Daten hinsichtlich der absorbierten Dosen zum Erzeugen von konkreten Bildern, die in der Einheit der zweidimensionalen Schnitte entlang einer vorher festgelegten Schnittebene umfasst sind, von "isodosierten" Oberflächen in drei Dimensionen, von durch die Planungssoftware der Radiotherapiebehandlung vorgeschlagenen Bildern.

10. Verfahren zum experimentellen Bestimmen einer Volumenverteilung von durch ein Phantom nach einem der Ansprüche 7 bis 9 empfangenen Strahlung bedingten Dosen, wobei die Strahlungen Partikel-Strahlungen sind, die in der Einheit umfasst sind, die aus Photonen, Elektronen, Protonen und schweren Kernen gebildet wird.

11. Radiometrisches physisches Phantom eines biologischen Organismus zum experimentellen Bestimmen, unter vorher festgelegten Bestrahlungsbedingungen, einer Volumenverteilung der durch das Phantom empfangenen Strahlungsdosen, die für die Dosisverteilung repräsentativ ist, die von dem biologischen Organismus, unter den gleichen Bestrahlungsbedingungen platziert, empfangen würde, wobei der biologische Organismus mindestens zwei biologische Gewebevolumina aufweist, von denen die chemischen Zusammensetzungen und die Dichten deutlich verschieden sind, **dadurch gekennzeichnet, dass** das physische Phantom umfasst
mindestens zwei in einem Trägerrahmen (342) in Position gehaltene Entladungsgefäße (302, 304, 308), durch jeweilige Wände umschlossen, die aus gegenüber Strahlung transparenter fester 3D-Druckertinte bestehen, und jeweils mit einem verschiedenen Dosimetrie-Gel (364, 366, 362) gefüllt sind,
wobei die mindestens zwei Entladungsgefäße (302, 304, 308) verschiedene und getrennte radiologische Volumen aufweisen, wobei jedes radiologische Volumen gleich oder sich durch Glätten von Konturen der Summe der Volumina einer vollständigen Einheit von zusammengefassten biologischen Geweben des radiologisch bestrahlten biologischen Organismus annähernd ist, und ausgehend von einem oder mehreren dreidimensionalen anatomischen Bildern der Gewebe hinsichtlich der chemischen Zusammensetzung und Dichten bestimmt ist, wobei die zusammengefassten Gewebe untereinander angrenzend sind und benachbarte chemische Zusammensetzungen und Dichten aufweisen, und
wobei die Dosimetrie-Gele (364, 366, 362), welche die Entladungsgefäße (302, 304, 308) füllen, jeweils physische Eigenschaften nahe an chemischen Zusammensetzungen und Dichten der zusammengefassten angrenzenden biologischen Gewebe aufweisen, deren Summe der Volumina gleich oder durch Glätten des radiologischen Volumens des Entladungsgefäßes, das das Gel füllt, angenähert ist.

12. Radiometrisches Phantom nach Anspruch 11, wobei
jedes Entladungsgefäß (302; 304; 308) mindestens eine Befüllungsöffnung zum Geleinlass und mindestens eine Befüllungsöffnung zum Luftauslass und mindestens zwei zwischen den Befüllungsöffnungen und dem Äußeren des Phantoms verbundene Befüllungsleitungen (332, 333; 334, 335; 338, 339) beinhaltet, und
für jedes Entladungsgefäß (302; 304; 308) die Anzahl an Befüllungsöffnungen des Geleinlasses, die Anzahl an Befüllungsöffnungen des Luftauslasses, die Lagen und die Durchmesser der Befüllungsöffnung(en) des Geleinlasses und der Befüllungsöffnung(en) des Luftauslasses, sowie die Strecken und die Durchmesser der Befüllungsleitungen des Entladungsgefäßes, die zwischen den Befüllungsöffnungen und dem Äußeren des Phantoms verbunden sind, konfiguriert sind, um das Befüllen des Entladungsgefäßes mit einem vorher festgelegten Volumenstrom ohne das Erzeugen von Volumenblasen zu erlauben.

13. System zur Herstellung eines radiometrischen physischen Phantoms, das Absorptions- oder Diffusionseigenschaften einer Strahlung eines biologischen Organismus simuliert, wobei der biologische Organismus mindestens zwei Volumina an biologischem Gewebe aufweist, deren chemischen Zusammensetzungen und Dichten deutlich verschieden sind,
wobei das System umfasst
ein Mittel zum Bereitstellen (404) eines oder mehrerer dreidimensionaler anatomischer Bilder der Gewebe hinsichtlich der chemischen Zusammensetzung und Dichten,
einen elektronischen Rechner (406), konfiguriert, um
ausgehend von einem oder mehreren dreidimensionalen anatomischen Bildern der Gewebe hinsichtlich der chemischen Zusammensetzung und Dichten ein dreidimensionales radiologisches Modell zu bestimmen (6), das repräsentativ für die Absorption und die Diffusion der Strahlung im Inneren des biologischen Organismus ist, durch Zusammenfassen der untereinander angrenzenden, und benachbarte chemische Zusammensetzungen und Dichten aufweisenden Gewebe in den radiologischen Organen,
wobei jedes radiologische Organ im Raum geometrisch ein radiologisches Volumen einnimmt, das gleich, oder durch Glätten der Konturen seiner Hülle der Summe der Volumina aller zusammengefassten Gewebe in dem gleichen radiologischen Organ annähernd ist, und durch eine radiologische Klasse gekennzeichnet ist, die den Bereich von den in dem gleichen radiologischen Organ zusammengefassten Geweben benachbarten chemischen Zusammensetzungen und Dichten identifiziert, und
ausgehend von dem dreidimensionalen radiologischen Modell die Ebenen eines materiellen Rahmenwerks des Phantoms als eine Einheit von Entladungsgefäßen, Gel-Befüllungsleitungen von Entladungsgefäßen und Trägerelementen zu bestimmen, um die Entladungsgefäße untereinander in Position zu halten, danach die Ebenen des materiellen Rahmenwerks in ein Steuerungsprogramm zum 3D-Druck zu überführen, auszuführen durch einen vorher festgelegten 3D-Drucker (408),
wobei der vorher festgelegte 3D-Drucker (408) konfiguriert ist, um das nicht mit Dosimetrie-Gel befüllte materielle Rahmenwerk des Phantoms mithilfe von Tinten zum 3D-Druck, die gegenüber Strahlung transparent sind, zu fertigen;
mindestens zwei verschiedene Dosimetrie-Gele (362, 346, 366), die dazu bestimmt sind, jeweils ein verschiedenes radiologisches Entladungsgefäß, das mit einem hinsichtlich der Klasse von chemischen Zusammensetzungen und Dichten verschiedenen radiologischen Organ assoziiert ist, zu befüllen, und
ein Mittel zum Befüllen (410) des leeren Phantoms durch die mindestens zwei Dosimetrie-Gele.

14. System zur Herstellung eines radiometrischen physischen Phantoms nach Anspruch 13, wobei der elektronische Rechner (406) für jede Zusammenfassung von biologischen Geweben in ein radiologisches Organ konfiguriert ist,
ein zwischenliegendes radiologisches Volumen als gleich der Summe der zusammengefassten Gewebevolumina in dem radiologischen Organ zu bestimmen, und
das radiologische Volumen des radiologischen Organs durch Glätten der Konturen des zwischenliegenden radiometrischen Volumens zu bestimmen, um die Einbuchtungen einzugrenzen und das zukünftige Befüllen des Dosimetrie-Gels zu erleichtern.

15. System zur Herstellung eines radiometrischen physischen Phantoms nach einem der Ansprüche 13 bis 14, wobei der elektronische Rechner (406) für jedes radiologische Organ konfiguriert ist,
die Anzahl an Befüllungsöffnungen des Geleinlasses, die Anzahl an Befüllungsöffnungen des Luftauslasses, jede Lage und jeden Durchmesser der Befüllungsöffnung(en) des Geleinlasses, jede Lage und jeden Durchmesser der Befüllungsöffnung(en) des Luftauslasses, sowie die Strecken und die Durchmesser der Befüllungsleitungen der Volumina der radiologischen Organe, die zwischen den Befüllungsöffnungen und dem Äußeren des Phantoms verbunden sind, zu bestimmen
um das Befüllen des radiologischen Organs ohne Volumenblasen zu erlauben.

16. System zur Herstellung eines radiometrischen physischen Phantoms nach Anspruch 15, wobei der elektronische Rechner (406) konfiguriert ist, um die Gesamtanzahl an Befüllungsöffnungen, die Lagen der Befüllungsöffnungen und die Strecken der Befüllungsleitungen auf eine Art und Weise zu bestimmen, dass die Leitungen die Ausbreitung der Strahlung zu einer zu behandelnden Zone in Abhängigkeit von der Lage der zu behandelnden Zone, des Eintrittswinkels des Strahlenbündels nicht stören.

17. System zur Herstellung eines radiometrischen physischen Phantoms nach Anspruch 16, wobei der elektronische Rechner (406) konfiguriert ist, um auch die geglätteten Formen der Volumina der radiologischen Organe zu bestimmen, um die Ausbreitung der Strahlung zu einer zu behandelnden Zone in Abhängigkeit von der Lage der zu behandelnden Zone, des Eintrittswinkels des Strahlenbündels nicht signifikant zu verändern.

18. System zum experimentellen Bestimmen einer Volumenverteilung der durch das Phantom empfangenen Strahlungsdosen, die repräsentativ ist für eine Dosisverteilung, die durch den Teil oder die Gesamtheit eines unter den gleichen vorher festgelegten Bestrahlungsbedingungen platzierten biologischen Organismus empfangen würde, umfassend:
ein System zur Herstellung (402) eines radiometrischen physischen Phantoms, gefüllt mit nach einem der Ansprüche 13 bis 17 definierten Dosimetrie-Gelen;
ein Bestrahlungsmittel (504) des physischen Phantoms (352), durch eine Strahlung unter vorher festgelegten Bestrahlungsbedingungen;
ein Mittel zum Auslesen (506) eines physischen Parameters der Gele in situ in dem Phantom, wenn letzteres, befüllt mit den bestrahlten Gelen, in ein Bildgebungsgerät platziert wird, das zum Beispiel ein Verfahren durch optische Übertragung nutzt, oder in ein NMR-Bildgebungsgerät, und Kodiermittel des ausgelesenen physischen Parameters in Form von Ausleserohdaten;
ein Behandlungsmittel (508) der Ausleserohdaten zum Bestimmen eines Bilds der dreidimensionalen Verteilung von in dem biologischen Organismus empfangenen Strahlungsdosen.

19. Computerprogrammprodukt, eine Anweisungseinheit beinhaltend, die, wenn sie durch den jeweils in den Ansprüchen definierten elektronischen Rechner (406) und/oder das Behandlungsmittel (508) der Ausleserohdaten des Systems zur Herstellung (402) und des Systems zum experimentellen Bestimmen (502) geladen und ausgeführt wird, einen Teil der Schritte des Verfahrens zur Herstellung nach Ansprüche 1 bis 6 und in dem experimentellen Bestimmungsverfahren, nach Ansprüche 7 bis 10, insbesondere die Schritte (6), (8), (106) und (108) durchführt.

## Claims

1. Method of producing a radiometric physical phantom of a biological organism in order to experimentally determine under predetermined conditions of irradiation a volume distribution of the doses of radiation received by the phantom, representative of the distribution of doses that would be received by the biological organism placed in the same conditions of irradiation, the biological organism having at least two volumes of biological tissues of which the chemical compositions and the densities are markedly different,
the method of producing comprising the steps consisting in:
from one or several anatomical three-dimensional images of the tissues in terms of chemical compositions and densities, determining (6) a radiological three-dimensional model, representative of the absorption and of the diffusion of the radiation inside the biological organism, by grouping together into radiological organs the mutually adjacent tissues and having chemical compositions and densities which are similar,
each radiological organ geometrically occupying in space a radiological volume, equal or approaching via smoothing the contours of its envelope the sum of the volumes of all of the tissues grouped into the same radiological organ, and being **characterised by** a radiological class identifying the range of the chemical compositions and densities which are similar to the tissues grouped into the same radiological organ; then
from the radiological model, carrying out (10) using 3D printing, a material framework of the physical phantom by reproducing each radiological volume by an enclosure filled with air and delimited by a wall, the wall consisting of a 3D printing ink; then
filling (16) each enclosure of the material framework of the phantom, associated with a radiological organ, by a dosimetric gel of which the physical characteristics are close or identical to those of the class of the chemical compositions and densities of the radiological organ.

2. Method of producing a radiometric physical phantom according to claim 1, wherein, during the determination (6) of the radiological model,
for each grouping of biological tissues in the same radiological organ,
an intermediate radiological volume is firstly determined as being equal to the sum of the volumes of the grouped tissues in the radiological organ, then
the radiological volume of the radiological organ is determined by smoothing the contours of the intermediate radiological volume in order to limit the crevices and facilitate the filling with dosimetric gel.

3. Method of producing a radiometric physical phantom according to claim 2, further comprising a step (8) of preparing software plans for the manufacture of the material framework of the phantom and translation of these plans into a framework 3D printing commands program, inserted between the step (6) of determining the radiological model and the step (10) of 3D printing of the framework of the phantom, wherein step of preparing (8),
for each enclosure associated with a radiological organ, the number of gel inlet filling orifices, the number of air outlet filling orifices, the locations and the diameters of the gel inlet filling orifice or orifices and of the air outlet filling orifice or orifices, as well as the paths and the diameters of the filling ducts of the enclosure, connected between the filling orifices and the outside of the phantom, are determined in such a way
as to allow for the filling of the radiological organ without bubbles of volume.

4. Method of producing a radiometric physical phantom according to claim 3, wherein the total number of filling orifices, the locations of the filling orifices and the paths of the filling ducts of all of the enclosures are determined so as to not hinder the propagation of the radiation to a zone to be treated according to the location of the zone to be treated and/or the inlet angle of the radiation beam.

5. Method of producing a radiometric phantom according to claim 4 in the smoothed forms of the volumes of the enclosures are also determined so as to not hinder the propagation of the radiation to a zone to be treated according to the location of the zone to be treated and/or the inlet angle of the radiation beam.

6. Method of producing a radiometric phantom according to any of claims 1 to 5 wherein the dosimetric gels are comprised in the set formed by the Fricke gels and the polymer gels.

7. Method of experimentally determining the volume distribution of the doses of radiation received by a radiometric physical phantom representative of a distribution of doses that would be received by the portion or by the totality of a biological organism placed in the same predetermined irradiation conditions, comprising the steps consisting in:
Supplying (104) a physical phantom filled with its dosimetric gels and manufactured according to any of claims 1 to 6;
Subsequently irradiating (106) the physical phantom under the predetermined irradiation conditions;
Proceeding (108) with a reading (110) of a physical parameter of the gels in situ in the phantom by placing the phantom filled with irradiated gels in an imaging device using for example a method via optical transmission or in an NMR imaging device and encoding (112) it in the form of raw data readings;
Subsequently processing (114) the raw reading data in order to determine an image of the three-dimensional distribution of the doses of radiation received in the portion or the totality of the biological organism.

8. Method of experimentally determining the volume distribution of the doses of radiation received by a physical phantom according to claim 7, **characterised in that** the raw reading information represents a read physical parameter comprised in the set formed by an optical density, a relaxation time.

9. Method of experimentally determining the volume distribution of the doses of radiation received by a physical phantom according to one of claims 7 and 8, wherein the step of processing the reading data (114) comprises the steps consisting in
Translating (116) the raw reading information in terms of absorbed doses; and
Grouping together (118) the translated data in terms of absorbed doses in order to create particular images comprised in the set of the two-dimensional cross-sections according to a predetermined cross-section plane, of the "isodose" surfaces in three dimensions, images proposed by radiotherapy treatment scheduling software.

10. Method of experimentally determining a volume distribution of doses due to the radiation received by a phantom according to one of claims 7 to 9, wherein the radiation is radiation of particles comprised in the set formed by the photons, the electrons, the protons, the heavy nuclei.

11. Radiometric physical phantom of a biological organism for experimentally determining under predetermined conditions of irradiation a volume distribution of the doses of radiation received by the phantom representative of the distribution of the doses that would be received by the biological organism placed in the same conditions of irradiation, the biological organism having at least two volumes of biological tissues of which the chemical compositions and the densities are markedly different, **characterised in that** the physical phantom comprises
at least two enclosures (302, 304,308), maintained in position in a support frame (342), delimited by respective walls consisting of a solid 3D printing ink that is transparent to radiation, and each one filled with a different dosimetric gel (364, 366, 362),
the at least two enclosures (302, 304, 308), having different and separated radiological volumes, each radiological volume being identical or close through smoothing of the contours to the sum of the volumes of a complete set of grouped biological tissues of the biological organism radioactively imitated and determined from one or several anatomical three-dimensional images of the tissues in terms of chemical compositions and densities, said grouped tissues being adjacent between them and having chemical compositions and densities which are similar, and
the dosimetric gels (364, 366, 362), filling said enclosures (302, 304, 308) each one having physical characteristics close to the chemical compositions and densities of the grouped adjacent biological tissues of which the sum of the volumes is identical or is approached via smoothing of the radiological volume of the enclosure that the gel is filling.

12. Radiometric phantom according to claim 11, wherein
each enclosure (302; 304; 308) presents at least one gel inlet filling orifice and at least one air outlet filling orifice, and at least two filling ducts (332,333; 334, 335; 338, 339) connected between the filling orifices and the outside of the phantom, and
for each enclosure (302; 304; 308), the number of gel inlet filling orifices, the number of air outlet filling orifices, the locations and the diameters of the gel inlet filling orifice or orifices and of the air outlet filling orifice or orifices, as well as the paths and the diameters of the filling ducts of the enclosure, connected between the filling orifices and the outside of the phantom, are configured to allow for the filling of the enclosure at a predetermined flow rate without creating bubbles of volume.

13. System for producing a radiometric physical phantom simulating the absorption or diffusion properties of a radiation of a biological organism, the biological organism having at least two volumes of biological tissues of which the chemical compositions and the densities are markedly different,
the system comprising
a means of supplying (404) one or several anatomical three-dimensional images of the tissues in terms of chemical compositions and densities,
an electronic calculator (406) configured to,
from one or several anatomical three-dimensional images of the tissues in terms of chemical compositions and densities, determine (6) a radiological three-dimensional model, representative of the absorption and of the diffusion of the radiation inside the biological organism, by grouping together into radiological organs the mutually adjacent tissues and having chemical compositions and densities which are similar,
each radiological organ geometrically occupying in space a radiological volume, equal or approaching, via smoothing the contours of its envelope, the sum of the volumes of all of the tissues grouped into the same radiological organ, and being **characterised by** a radiological class identifying the range of the chemical compositions and densities which are similar to the tissues grouped into the same radiological organ, and
from the radiological three-dimensional model, determine the plans of a material framework of the phantom as being a set of enclosures, of ducts for filling enclosures with gel, and of support elements for maintaining the enclosures in position between them, then translate the plans of the material framework into a program of 3D printing commands to be executed by a predetermined 3D printer (408),
the predetermined 3D printer (408) configured to carry out the material framework of the phantom not filled with dosimetric gels, using 3D printing inks that are transparent to radiation;
at least two different dosimetric gels (362, 364, 366) intended to each fill a different radiological enclosure associated with a different radiological organ in terms of chemical compositions classes and densities, and
a means of filling (410) the empty phantom with at least two dosimetric gels.

14. System for producing a radiometric physical phantom according to claim 13, wherein the electronic calculator (406) is configured for each grouping of biological tissues in a radiological organ,
Determining an intermediate radiological volume as being equal to the sum of the volumes of the tissues grouped in the radiological organ, and
Determining the radiological volume of the radiological organ by smoothing the contours of the intermediate radiological volume so as to limit the crevices and facilitate the future filling of the gel.

15. System for producing a radiometric physical phantom according to any of claims 13 to 14, wherein the electronic calculator (406) is configured for each radiological organ,
Determining the number of gel inlet filling orifices, the number of air outlet filling orifices, each location and diameter of the gel inlet filling orifice or orifices, each location and diameter of the air outlet filling orifice or orifices, as well as the paths and the diameters of the filling ducts of the volumes of the radiological organs, connected between the filling orifices and the outside of the phantom, in such a way
as to allow for the filling of the radiological organ without bubbles of volume.

16. System for producing a radiometric physical phantom according to claim 15, wherein the electronic calculator (406) is configured to determine the total number of filling orifices, the locations of the filling orifices and the paths of the filling ducts in such a way that the ducts do not hinder the propagation of the radiation to a zone to be treated according to the location of the zone to be treated, to the inlet angle of the beam of radiation.

17. System for producing a radiometric physical phantom according to claim 16, wherein the electronic calculator (406) is configured to also determine the smoothed shapes of the volumes of the radiological organs in order to not significantly modify the propagation of the radiation towards a zone to be treated according to the location of the zone to be treated, to the inlet angle of the beam of radiation.

18. System for experimentally determining a volume distribution of doses of radiation received by a phantom representative of a distribution of doses that would be received by the portion or by the totality of a biological organism placed in the same predetermined conditions of irradiation, comprising:
A system for producing (402) a radiometric physical phantom filled with dosimetric gels defined according to any of claims 13 to 17;
A means of irradiation (504) of the physical phantom (352) by a radiation under predetermined conditions of irradiation;
A means of reading (506) a physical parameter of the gels in situ in the phantom when the latter, filled with irradiated gels, is placed in an imaging device that uses for example a method via optical transmission or in an NMR imaging device and a means for encoding the read physical parameter in the form of a raw reading data;
A means for processing (508) the raw reading data in order to determine an image of the three-dimensional distribution of the doses of radiation received in the biological organism.

19. Computer program product presenting a set of instructions, which when they are loaded and executed by the electronic calculator (406) and/or the means of processing (508) the raw reading data of the system for producing (402) and of the system for experimentally determining (502) defined respectively in the claims implementing a portion of the steps of the method for producing defined according to claims 1 to 6 and in the method for experimentally determining defined according to claims 7 to 10, in particular the steps (6), (8), (106) and (108).
